# EUROPEAN PATENT APPLICATION

(11) **EP 2 412 290 A1**
(43) Date of publication of application: **01.02.2012**
(21) Application number: 10171524.1
(22) Date of filing: 30.07.2010
(51) Int. Cl.: A61B 1/00, A61B 1/04, A61B 1/05

(54) **Endoscope and endoscope system**

(71) Applicant: Flegler, Stephan, 79576 Weil am Rhein (DE); Krüger, Colin, 12209 Berlin (DE)
(72) Inventor: Flegler, Stephan, 79576 Weil am Rhein (DE); Krüger, Colin, 12209 Berlin (DE)
(74) Representative: Herrmann, Franz

(57) **Abstract**

An endoscope (4) comprises video units (21) that are disposed at the distal end (5) of a shaft (19) of the endoscope (4) and that can be splayed out from the shaft (19) for examining a cavity. The endoscope can be used for generating stereoscopic views of the cavity. A corresponding endoscope system comprises a display for displaying three-dimensional view of the cavity.

## Description

The invention relates to an endoscope for examining a cavity comprising:
- an elongated shaft having a distal end and a proximal end and a longitudinal axis extending from the proximal end to the distal end;
- at least two video units, the distance between which is variable and among which at least one movable video unit is pivotably attached to the shaft by a link arranged between the proximal end of the at least one movable video unit and the shaft;
- actuating elements arranged for moving the at least one movable video unit in a lateral direction with respect to the longitudinal axis of the shaft from a first position, that is used for introduction and withdrawal of the endoscope into and out of a cavity to be examined, to a second position for examining the cavity.

The invention further relates to an endoscope system.

Such an endoscope is known from US 5,166,787 A. The known endoscope has a video unit arranged at the distal end of the endoscope shaft. The video unit comprises a lens, through which incident light can reach an image recorder disposed behind the lens. The video unit is held in such a moveable manner at the endoscope shaft that the outer contour of the cross-section of the video unit is located essentially within the outer contour of the cross-section of the distal end of the endoscope shaft when being introduced into the cavity to be examined. After the introduction procedure has been terminated, the video unit can be rotated around an excentric rotation axis, which extends in parallel to the longitudinal axis of the shaft. After the rotational movement has been finished, the outer contour of the cross-section of the video unit extends beyond the cross-section of the shaft. The known endoscope can also be provided with two video units that can be pivoted around a common rotational axis extending in parallel to the longitudinal axis of the shaft resulting in video units disposed in an excentric way with respect to the shaft of the endoscope. Both video units can be used for recording stereoscopic images.

One drawback of the known endoscopes is that the endoscopes cannot be withdrawn immediately in a case of emergency since the video unit must first be rotated back to the initial position before the endoscope can be withdrawn. Another disadvantage is the location of the entrance lenses of the video units that are disposed at the outside of the endoscope if the endoscope is introduced into the cavity. During the introduction process, these lenses might be damaged.

A further endoscope is known from US 2002/0007110 A1. The known endoscope comprises a shaft at the distal end of which a solid state image-recording system is disposed, which is designed in a manner of lateral-view optics. The endoscope shaft is deflectable in the distal region in such a manner that the viewing direction of the solid state image-recording system is approximately parallel to the longitudinal axis of the proximal part of said endoscope shaft.

This endoscope also cannot easily be withdrawn from the cavity since moving the deflected image recording system back to its initial position requires a considerably amount of space. Additionally, the optical lenses of the image-recording system are located at the outside in the initial position so that these lenses can also be damaged while introducing the endoscope into the cavity.

From JP 2006-187385 A an endoscope device is known that is arranged for recognizing a distance and a contact position between an inserting instrument or a treating instrument and an object. For this purpose, stereo measurements are performed for obtaining three-dimensional information on a measurement point by evaluating video images obtained from two left and right video units. The control unit may use the three-dimensional information for obtaining the contact position at the route of the treating instrument. Thus, the three-dimensional relation of the treating instrument and the object within a cavity to be examined can be recognized.

JP 2003-199707 A describes a method and an apparatus to align an observation image obtained by an optical system of an endoscope apparatus and a tomographic image corresponding to the observation image. The known method and apparatus evaluates markers that are attached to the proximal end of the endoscope in order to determine the position of the endoscope.

US 6,028,672 A gives an overview on various methods for determining three-dimensional surface profiles.

Proceeding from this related art, the present invention seeks to provide a safe endoscope and endoscope system that are easy to handle.

This object is achieved by an endoscope and an endoscope system having the features of the independent claim. Advantageous embodiments and refinements are specified in claims dependent thereon.

The endoscope comprises at least one movable video unit that is pivotably attached to the shaft by a link between the at least one movable video unit and the shaft. The at least one movable video unit can be moved in a lateral direction with respect to the longitudinal axis of the shaft from a first position, that is used for introduction and withdrawal of the endoscope into and out of a cavity to be examined, to a second position for examining the cavity. In the first position, an optical entrance window of the at least one movable video unit faces the inside of the shaft. In the second position, the at least one movable video unit is splayed out from the shaft by a pivoting motion, which transfers the at least one movable video unit from the first position to the second position. The pivoting motion is performed around an pivot axis oriented in a transverse direction with respect to the longitudinal axis of the shaft and which increases the angle between the longitudinal axis of the shaft and a plane that is perpendicular to the line of sight of the at least one movable video unit.

Thus, the at least one movable video unit of this endoscope has an optical entrance window that is oriented towards the inside of the shaft while the movable video unit is in its first position. In this position, the optical entrance window of the at least one movable video unit extends along the longitudinal axis of the shaft and is protected by the outside of the video unit. Consequently, the optical entrance window is prevented from any damage during the introduction process. It can also easily be withdrawn from the cavity since the splayed out video unit will be forced to return to its first position once the endoscope is withdrawn through the trocar leading into the cavity or any other opening of the cavity.

It should be noted that the video units may also be arranged for taking individual fixed images. The optical entrance window should further be understood as being the opening in a housing of the video unit that is generally covered by a transparent optical element, through which images can be taken. The optical axis of each the video units coincides with the central ray of the optics of the video unit.

In one embodiment, the endoscope is provided with at least one stationary video unit, whose position with respect to the shaft is unchangeable and which is disposed at the distal end of the shaft. Such a stationary video unit can be used for controlling the introduction process of the endoscope.

In most cases, the video units are disposed at the same position along the longitudinal axis of the shaft in the first and/or second position of the at least one movable video unit. Such an arrangement of the video units has the advantage that the entrance windows of the video units may be located on a common plane in the second position which simplifies the geometry that must be taken into account while generating three-dimensional views based on the images taken by the video elements.

In a further embodiment, the optical entrance windows of the at least one movable video unit faces a lateral side of the shaft in the first position of the at least one movable video unit, which offers additional protection against any damage.

For obtaining sufficient space between the video units, at least two video units are movable and pivotably attached to the shaft by links between the at least two movable video units and the shaft. These at least two video units perform the same sort of motion from the first to the second position as the at least one movable video unit.

Since the optical entrance windows of the at least two movable video units are facing the inside of the endoscope in their first position, the optical entrance windows may generally facing each other in the first position of the movable video units.

In the case of at least two movable video units, the pivoting motions of the video units are performed around an associated pivot axis wherein the pivot axes are different. Thus, a large distance between the video units can be obtained in the second position.

The actuating elements usually comprise a stop against which a counterpart abuts in the second position for adjusting the position of the at least one movable video unit. For a smooth bending of the link between the movable video units and the shaft of the endoscope, the link between the at least one movable video unit and the shaft may contain more than one articulation.

The link between the at least one movable video unit extends between the proximal end of the at least one movable video unit and the shaft providing a smooth transition from the shaft to the video unit.

Besides the generation of three-dimensional view of the cavity, the endoscope may also be used to retrieve spatial coordinates of the cavity. Thus, the shaft and/or any one of the video units is provided with a component for retrieving spatial coordinates on the cavity to be examined. This component can be a semiconductor sensor chip that is arranged to measure the time of flight of a light signal emitted by a light source of the endoscope and reflected back to the sensor chip by a surface within the cavity. This component may also be a projector that is arranged for projecting a pattern on a surface within the cavity. Images of the pattern on the surface of the cavity can then be taken by the video unit and evaluated, resulting in a measured profile of the pattern. Besides these two methods any other method for measuring distances and for determining spatial coordinates might be considered by the skilled person.

In one further embodiment, the at least one movable video unit covers the distal end of the endoscope in the first position only partially allowing a entrance window or a component for determining distances to be placed on the uncovered portion of the distal end of the endoscope so that the entrance window or component can be used for controlling the introduction process.

The endoscope described herein may also be used for an endoscope system comprising a video processing unit arranged to be connected to at least two video units of an endoscope and a display connected with the video processing unit and arranged for displaying a three-dimensional view of the cavity to be examined.

Such an endoscope system usually comprises an autostereoscopic display, a holographic display or a display system that uses a binocular filter device for conveying a three-dimensional impression.

The endoscope system can further be provided with a data reduction unit for retrieving spatial coordinates of the cavity to be examined wherein the data reduction unit is arranged to process images recorded by the video units and/or additional data provided by further components located in the video units and/or the shaft of the endoscope. As mentioned above, the further component can be a sensor chip arranged for measuring the time of flight of a light signal or projector for projecting a pattern. In the latter case, the data reduction unit may also evaluate images taken by the video units. Another possibility is the use of markers fixed to particular locations within the cavity. The position of these markers may be determined by triangulation since the known distance between the video units may be used as a base and since the angles can be determined from the images taken by the video units.

The data obtained from the data reduction unit can further be used for determining the present location, orientation, and/or dimensions of an object located within the cavity or limiting the cavity and for adapting a three-dimensional model of the object, which has be obtained using another modality, to the present location, orientation, and/or dimensions of the object. The model of the object can then be merged with the three-dimensional representation of the object obtained by the endoscope. An example of such a representation would be a grid model of an outer contour of an organ, wherein the outer contour of the organ has been determined previously by an apparatus for computer tomography or any other modality for generating three dimensional representations of components inside the body of a patient.

Further advantages and properties of the present invention are disclosed in the following description, in which exemplary embodiments of the present invention are explained in detail based on the drawings:
- Figure 1: gives an overview on an endoscope system;
- Figure 2: is a view of the distal end of an endoscope in the closed state;
- Figure 3: is a lateral view of the endoscope from Figure 2 in the closed state;
- Figure 4: is a view of the distal end of the endoscope from Figures 2 and 3 in an open state;
- Figure 5: is a lateral view of the endoscope from Figures 2 to 4 in the open state;
- Figure 6: is a longitudinal section depicting some internal components of the endoscope from Figure 2 to 5;
- Figure 7: is a view of the distal end of a further embodiment of an endoscope;
- Figure 8: is a lateral view of the endoscope from Figure 7 in a closed and an open state;
- Figure 9: is a view of the distal end of another embodiment of the endoscope in a closed state;
- Figure 10: is a view of the distal end of the endoscope from Figure 9 in an open state;
- Figure 11: is a front view of another embodiment of an endoscope in the closed state;
- Figure 12: is a front view of the endoscope from Figure 11 in the open state;
- Figure 13: is a view of a longitudinal section of the embodiment shown in Figure 11 and 12; and
- Figure 14: is a block diagram of an evaluation circuit of images and data taken with any of the endoscope shown in the previous Figures.

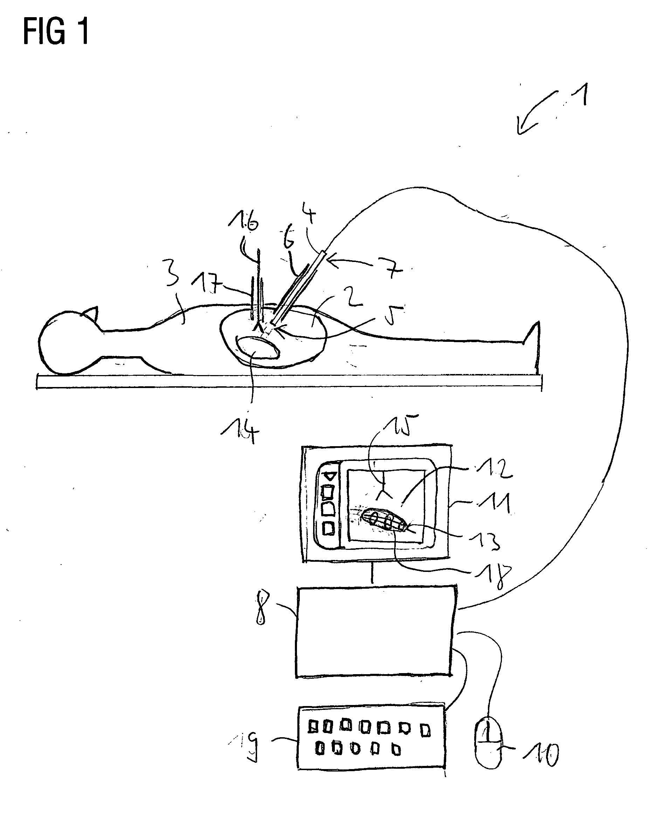

Figure 1 gives an overview on an endoscope system 1 that can be used for examining a cavity 2 within the body of a patient 3. The endoscope system is provided with an endoscope 4, whose distal end 5 can be introduced into the cavity 2 via a trocar 6. At a proximal end 7, the endoscope 4 is connected to an data processing unit 8, that might be a commercial computer comprising a data input device 9 and a pointing device 10. The data processing unit 8 is further connected to a display 11, on which a view 12 of the cavity 2 can be displayed. The view 12 represents the cavity 2 as seen from the distal end 5 of the endoscope. In Figure 1 the view 12 shows a representation 13 of an organ 14 located in front of the distal end 5 of the endoscope 4. The view 12 further contains an image 15 of a surgical instrument 16 that has been introduced into the cavity 2 through another trocar 17 and that might be used for treating the organ 14.

The view 12 can be an autostereoscopic view of the cavity 2 or a representation that needs a binocular device for generating a three-dimensional impression of the cavity 2. The binocular device may contain shutters synchronized with the view 12 displayed on the display 11 or glasses with different colors. The skilled person may consider various ways for generating a three-dimensional view of the cavity 2.

It should also be noted that the representation 13 of the organ 14 is complemented by a grid model 18 that is obtained from another modality, for example an apparatus for computer tomography or any other medical device that is capable of generating three-dimensional data of the body of the patient 3, from which the model 18 can be reconstructed.

For generating three-dimensional views of the cavity 2, the endoscope 4 must be capable of viewing the cavity 2 from at least two different positions.

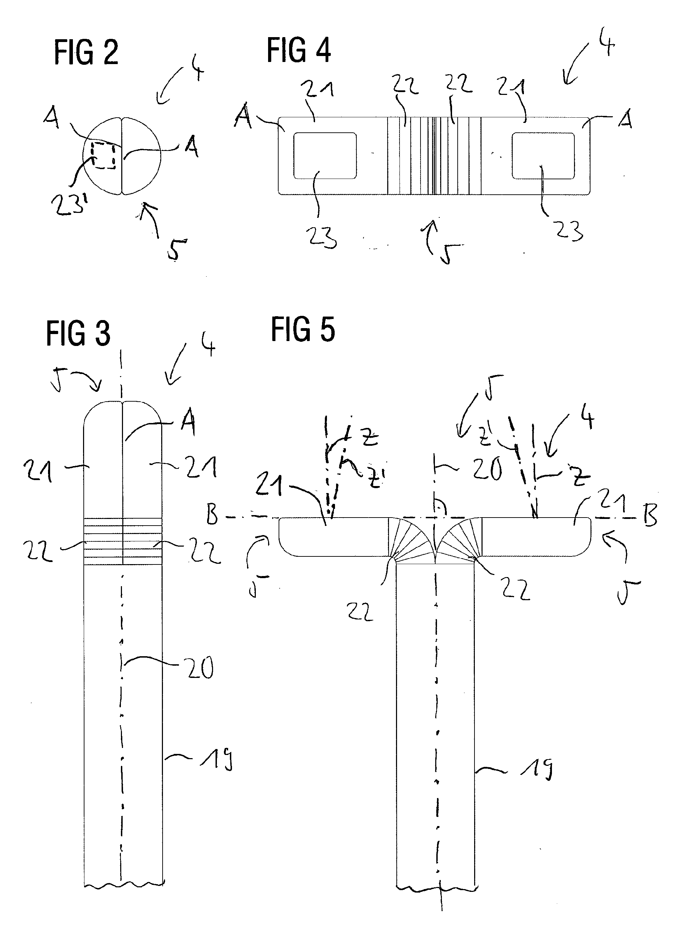

Figure 2 to 5 show a first embodiment of the endoscope 4 that might be used for the endoscope system 1 depicted in Figure 1. Figure 2 and 3 show the embodiment of the endoscope 4 in the closed state, whereas Figure 4 and 5 show the endoscope 4 in an open state. Figure 2 and Figure 4 each show a front view of the distal end 5 of the endoscope 4. Lateral views of the endoscope 4 are shown in Figures 3 and 5.

As can be seen from Figure 3 and 5, the endoscope 4 comprises an elongated shaft 19, that extends from the distal end 5 to the proximal end 7 and that has a longitudinal axis 20. At the distal end 5, the endoscope 4 comprises two video units 21, that are connected to the shaft 19 by bending sections 22. As can be recognized from Figure 4, the video units 21 are each provided with an optical entrance window 23, through which light can enter the video units 21. The optical entrance windows 23 are each disposed on a flat surface of the housing of the video units 21. These flat surfaces and the entrance windows 23 define planes A, that are perpendicular to optical axes Z of the video units 21. Thus, during the pivoting motion, that transfers the video units 21 from the first closed state to the second open state, the planes A are at first parallel to the longitudinal axis 20 of the shaft 19. During the pivoting motion, the angle between the longitudinal axis 20 and the planes A successively increases until the video units 21 reach their final position, in which the planes A assume an angle of 90° with respect to the longitudinal axis 20.

The video units 21 or the shaft 19 might also be provided with illumination sources, that can be used for illuminating the objects in front of the distal end 5 of the endoscope 4.

In the closed state, the video units 21 are aligned with the longitudinal axis 20 and the contour of the cross-section formed by the video units 21 is approximately within the contour of the cross-section of shaft 19.

In the open state, the bending section 22 is deflected in an outward or lateral direction in a manner that the video units 21 are splayed out from the shaft 19. In the open state, the video units 21 are oriented in a direction perpendicular to the longitudinal axis 20. Thus, the entrance windows 23 are located in the same common plain B, which facilitates the generation of the three-dimensional view due to the simple geometry. In principle, however, it may also be possible, to deflect the video units 21 by an angle smaller than 90° or more than 90° as long the area of view of both video units 21 overlap.

As shown in Figure 2, one of the video units 21 may also be provided with an entrance window 23', which is located at the distal end 5 of the video unit 21 and whose optical axis is aligned with the longitudinal axis 20 in the closed state. The entrance window 23' may be necessary for controlling the introduction of the endoscope 4 into the cavity 2. If there is not enough space at the distal end 4 of the video unit 21 for providing the entrance window 23', the distal end of the corresponding video unit 21 may extend beyond the distal end 5 of the other video unit 21. The portion of the video unit 21, which extends beyond the distal end 5 of the other video unit 21 may extend into the cross section of the other video unit 21 or cover the other video unit 21 in the closed state. In such an embodiment, the video units 21 would be asymmetrically shaped, but at the distal end 5 of the video unit 21 extending beyond the distal end 5 of the other video unit 21, there would be enough space for providing the entrance window 23'.

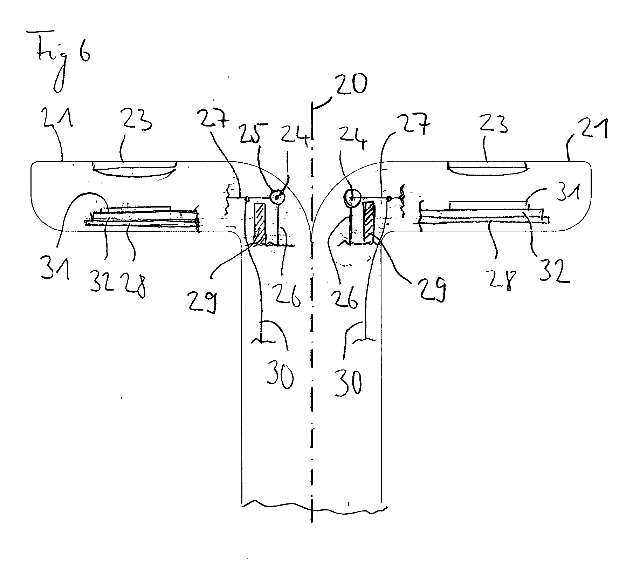

In any case, the video units 21, that are transferred from a first closed state to a second open state, must assume a defined position, in which the spatial relation between the entrance windows 23 is known, unless there are motion sensors or position sensors, which allow for determining the opening angle of each of the video units 21. Figure 6 illustrates in a schematic way the mechanical construction of the bending section 22 and the optical elements contained in the video unit 21. The mechanics of the bending section 22 comprises essentially a pivot axis 24 that might be realized by a link having one or more articulations.

In Figure 6 the basic structure of the mechanics is illustrated by a hinge 25 that connects in a pivotable manner a support 26, that forms part of the shaft 19, and a lever 27, that is attached to a support structure 28 of the video unit 21. The mechanics of the bending section 22 further comprises stops 29, against which the lever 27 abuts, thus bringing the video units 21 in a defined spatial interrelation in the open position.

It should be noted that the stops 29 might be also arranged such that the angle between the front sides A and the longitudinal axis 20 is less than 90°. The stops may further be adjustable so that the angle between the front sides A of the video units 21 and the longitudinal axis 20 may vary. Thus the optical axes Z can intersect at a finite distance from the endoscope 4. If the angle between the front sides A and the longitudinal axis is chosen to be smaller than 90°, the optical axes will generally intersect along the longitudinal axis 20.

It should be noted, that the skilled person will find a multitude of ways for realizing the mechanics of the bending section 22 in practice. Figure 6 intends only to show the basic functionality of such a mechanism. In general, the mechanism will be actuated from a handle disposed at the proximal end 7 of the endoscope 4.

According to Figure 6, for actuating the bending section 22 a bowden cable 30 may be connected to the lever 27. An alternative for actuating the levers 27 may be a pneumatic or a hydraulic device inside the shaft 19. The levers 27 may also actuated by a gear that synchonizes the movement of the levers such, that the levers 27 perform symmetric movements with respect to the longitudinal axis 20.

Figure 6 depicts also some optical elements of the video units 21. The entrance window 23 may also have the functionality of a lens that images an outside object on a semiconductor video chip 31 located on a printed circuit board 32 that is disposed on the support structure 28 of the video units 21. The video chip 31, that is located in the optical path behind the entrance window 21, might be a CCD chip or any other semiconductor device transforming light into electrical signals and that can be used for generating a two-dimensional picture. The optical axis Z of one video unit 21 equals the central ray of the optics of the video unit 21. In the embodiment shown in Figure 6, the optical axis is the symmetry axis of the entrance window 23. In a modified embodiment, the optics of the video unit 21 may also be integrated with the video chip. For instance, a video chip may also include optical elements such as microlenses for shortening the optical path. In another embodiment, the entrance window 23 may be have the shape of a Fresnel lens.

In the embodiment shown in Figure 2 to 6, the optical axes in the open state are shown to be aligned in parallel to the longitudinal axis 20. But the optical axes Z of the video units 21 may also intersect at an intersection point at a finite distance from the endoscope 4. For this purpose, the video chips may be disposed at an angle with respect to the front sides A resulting in inwardly inclined optical axes Z' as depicted in Figure 5.

It should be noted that the entrance windows 23 are facing each other in the closed state of the embodiment of the endoscope 4 shown in Figures 2 to 6. Thus, the entrance windows are protected against damaging during the introduction of the distal end 5 of the endoscope 4 through the trocar 6.

The embodiment shown in Figures 2 to 6 comprises two video units 21. Each of these video units 21 is provided with a single entrance window 23 and an associated video chip 31. In a modified embodiment, each of the video units is provided with more than one entrance window and more than one associated video chip, thus allowing multiple stereoscopic views from various viewing directions. Such multiple views may be necessary for the autostereoscopic display 11 so that the user can move his head and still gets an autostereoscopic view of the object. Such multiple views also allow multiple viewing perspectives of the object.

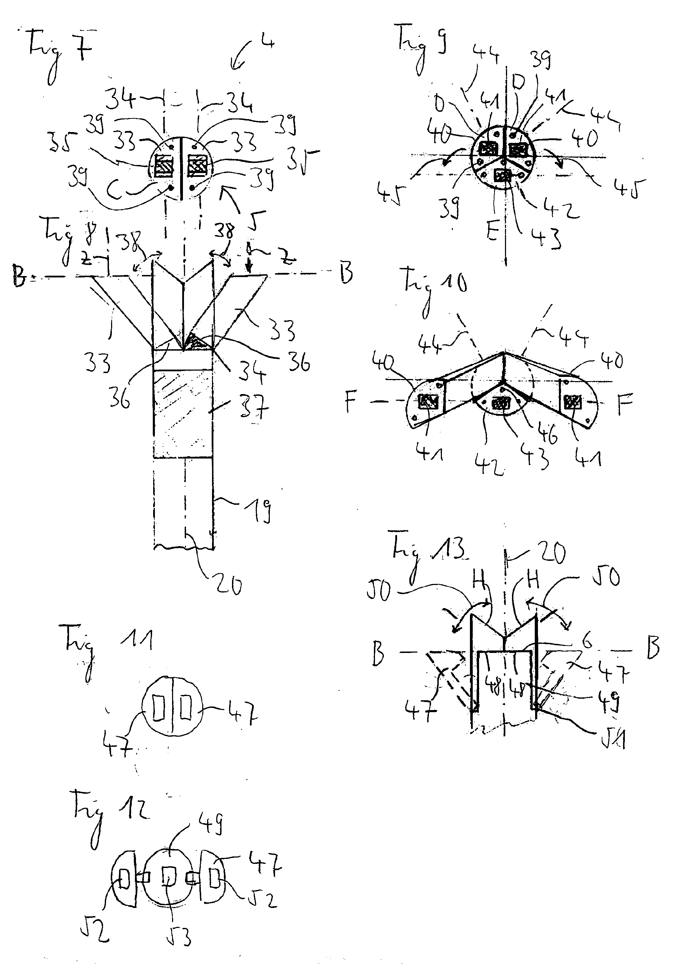

Figure 7 and 8 show another embodiment of the endoscope 4. Figure 7 shows a front view of the distal end 5 of the endoscope 4. The embodiment of the endoscope 4 depicted in Figure 7 and 8 has video units 33, that are provided with a slanted front side C, that is inclined towards the longitudinal axis 20 if the video units 33 are located side by side at the distal end 5 in the closed state. In the closed state, the slanted front side C form a V-shaped profile of the distal end 5 if the front sides C are viewed edge on. If the video units 33 are transferred in the open state by pivoting the video units 33 around a pivot axis 34, the front sides C are located in the common plain B, that is perpendicular to the optical axes Z of the entrance windows 35. The entrance windows 35, that are located on the front side C, are aligned in the common plain B, simplifying the geometry that has to be taken into account while generating the three-dimensional views 12.

As in the embodiment of the endoscope 4 shown in Figures 2 to 6, the embodiment of the endoscope 4 shown in Figure 7 and 8 is provided with bending sections 36 that are disposed between the proximal end of the video units 33 and the distal end of the shaft 19. Further towards the proximal end 7 of the shaft 19, the endoscope 4 from Figures 7 and 8 is provided with a shaft bending section 37, which allows varying the orientation of the common plane B. The mechanics inside the shaft 19 of the endoscope 4 displayed in Figure 7 and 8 must therefore be arranged for the pivoting motion 38 of the video units 33 and a bending motion of the shaft 19.

As in the embodiment shown in Figure 2 to 6, the angle between the planes C and the longitudinal axis 20 is increased by the pivoting motion 38 from the first closed state to the second open state. In the open state of the embodiment shown in Figures 2 to 6, the front sides C may assume an angle with respect to the common plane B, which is perpendicular to the longitudinal axis 20, in order to let the optical axes of the video units 33 intersect at a finite distance from the endoscope 4.

It should be noted, that the front side C may also be provided with illumination devices 39 for illuminating the cavity 2.

In Figure 9 and 10 a further embodiment of the endoscope 4 is shown, in which the distal end 5 of the shaft 19 is divided into three sections. Two of the sections are formed by movable video units 40, whose front side D is provided with entrance windows 41 and the illumination devices 39. The third section is formed by a stationary video unit 42, whose front side E is also provided with an entrance window 43.

The movable video units 40 are provided with bending sections similar to the bending section 22. The bending sections of this embodiment are provided with intersecting pivot axes 44. The video units 40 can now perform a pivoting motion 45 around intersecting pivot axes 44 such that, in the open state, the entrance windows 41 of the movable video units 40 and the entrance window 43 of the stationary video unit 42 are aligned along a line F and also located in the common plane B. The entrance window 43 can be used for observing the cavity 2 while introducing the endoscope 4 into the cavity 2. The view through the entrance window 43 can further be used for generating the three-dimensional view 12 of the cavity 2.

In the open state, the front sides D can also be aligned at an angle smaller 90° with respect to the central front side E in order to let the optical axes of the video units 40 and 42 intersect at a finite distance from the endoscope 4.

The stationary video unit 42 can also be provided with a measurement device 46 for retrieving spatial coordinates of the cavity 2.

Figure 11 to 13 show a modified embodiment of an endoscope having two movable video units 47. The video units 47 comprise a proximal recess 48 that covers a stationary video unit 49 that is located in a central portion of the shaft 19. The video units 47 can each perform a pivoting motion 50 around pivot axes 51 which are located in a distance from a distal front side G of the central portion 49. If the video units 47 reach their final position, slanted front sides H of the video units 47 are located in the common plain B together with the front side G of the central portion 49 of the shaft 19 resulting in aligned entrance windows 52 of the video units 47 and a central entrance window 53 of the central stationary video unit 49.

In the open state, the front sides H can also be aligned at an angle smaller 90° with respect to the central front side G in order to let the optical axes of the video units 47 and 49 intersect at a finite distance from the endoscope 4.

The embodiments shown in Figures 9 to 13 have the advantage that three video units are available so that two stereoscopic views form different viewing directions can be produced.

In modified embodiments of the endoscopes 4 shown in Figures 9 to 13, the central stationary video units 42 and 49 are arranged for recording optical images of the cavity 2, whereas one of the video units 40 or 47 is provided with the measurement device 46, which might in particular be a projector for projecting a pattern on surfaces within the cavity 2, which might be evaluated for generating three-dimensional profiles or three-dimensional coordinates of the cavity 2 using the images taken by the other video unit 40 or 47. The generation of the three-dimensional profiles or coordinates is based on the method of triangulation. If the distance between the projector and the video unit 40 or 47 and the direction, in which a particular pattern is projected, is known and if the viewing direction, from which the pattern is observed in the image recorded by the video unit 40 or 47, can be determined, the three-dimensional profiles or coordinates can be determined by triangulation. These profiles or coordinates can be assembled into a model of the objects seen from the endoscope.

In such an embodiment, multiple views of the object can be generated by the three-dimensional model resulting from the measurement and the optical view taken by the central stationary video unit 42 or 49.

It should be noted that the pattern can be projected inter-mittingly. In time intervals, in which the projection of the pattern is interrupted, the optical images can be recorded by the central video unit 42 or 49 and in time intervals, in which the pattern is projected, the video unit 40 or 47, can record the images that are needed for constructing a three-dimensional model of the cavity 2.

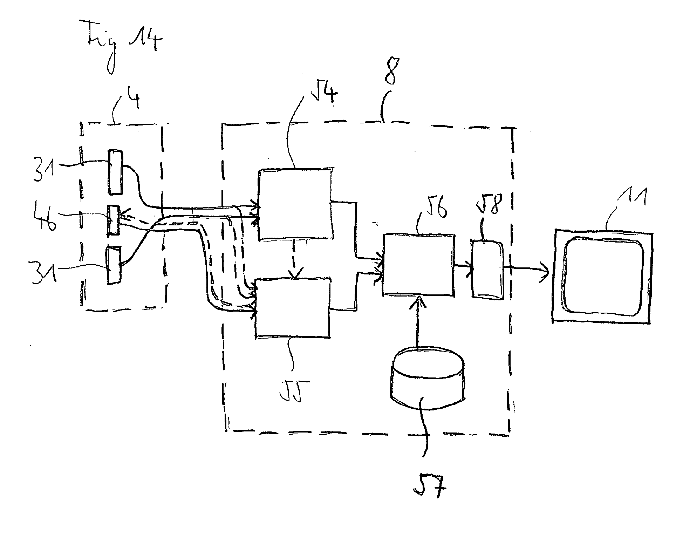

Figure 14 shows a block diagram of the data flow within the endoscope system 1 from Figure 1. The image data generated by the video chip 31 are transferred to the data processing unit 8 where the image data generated by the video chip 31 is processed in an image processing unit 54. The image processing unit generates a three-dimensional view 12 of the cavity 2 based on the image data generated by the video chip 31. As mentioned previously in connection with the embodiment shown in Figure 9 and 10, the endoscope 4 may also be provided with an measurement device 46 for measuring distances in particular for generating three-dimensional coordinates of the cavity 2 including objects contained in the cavity 2 or limiting the cavity. Such an object may be the organ 14 shown in Figure 1. This measurement device 46 might be a CMOS chip that is arranged for measuring the time of flight of a light signal emitted by one of the illumination units 39 and reflected back to the measurement device 46 by a surface within the cavity 2. The measurement device 46 may also be a projector that is arranged for projecting a pattern on surfaces within the cavity 2 which might be evaluated using the images generated by the video chip 31 for determining spatial coordinates of these surfaces.

In the following, we assume that the measurement device 46 is a CMOS-sensor for measuring distances. The data generated by the measurement device 46 are transferred to a data reduction unit 55 which generates spatial coordinates of the surface to be examined. The information generated by the image processing unit 54 and the data reduction unit 55 are supplied to an registration unit 56, that retrieves the three-dimensional model 18 of the viewed and measured object from a data base 57, in which the models 18 are stored, and registers the model 18 with the view 12. The three-dimensional model 18 of the object might be obtained using other modalities such as an apparatus for computer tomography or any other medical device capable of generating three-dimensional representations of the body of the patient 3.

Using the data of the data reduction unit 55, the location, orientation, and/or dimensions of the three-dimensional model 18 of the object can be fitted to the present location, orientation, and/or dimensions of representation 13 of the object. Thus the model 18 can be adapted to the present representation 13 object. Such a registration process is generally necessary since the location, orientation, and/or dimensions of an object within the body of the patient 3 change continuously due to the movements of the body and within the body.

Finally an image rendering unit 58 generates the display data that represent the view 12 containing the representation 13 obtained by the endoscope 4 and the model 18 obtained from another modality. These display data are finally provided to the display 11.

It should be noted that at least one marker may also be used for registering the view 12 with a model of the object. Such markers are preferably made from a material and provided with such a surface that the markers are visible in the image date generated by the other modality and in the images generated by the endoscope 4.

The endoscopes described herein have moveable video units that perform pivoting motions in opposite directions. In an alternative embodiment, only one video unit may be movable with respect to the shaft and arranged to be splayed out from the shaft. In the closed position of such an embodiment, the entrance window of the single movable video unit will generally face the shaft.

Throughout the description and claims of this specification, the singular encompasses the plural unless the context otherwise requires. In particular, where the indefinite article is used, the specification is to be understood as contemplating plurality as well as singularity, unless the context requires otherwise.

Features, integers, characteristics, compounds or groups described in conjunction with a particular aspect, embodiment or example of the invention are to be understood to be applicable to any other aspect, embodiment or example described herein unless incompatible therewith.

## Claims

1. An endoscope for examining a cavity (2) comprising:
- an elongated shaft (19) having a distal end (5) and a proximal end (7) and a longitudinal axis (20) extending from the proximal end (7) to the distal end (5);
- at least two video units (21, 33, 40, 42, 47, 49), the distance between which is variable and among which at least one movable video unit (21, 33, 40, 47) is pivotably attached to the shaft (19) by a link (22) arranged between the proximal end (7) of the at least one movable video unit (21, 33, 40, 47) and the shaft (19);
- actuating elements (25-30) arranged for moving the at least one movable video unit (21, 33, 40, 47) in a lateral direction with respect to the longitudinal axis (20) of the shaft (19) from a first position, that is used for introduction and withdrawal of the endoscope into and out of a cavity (2) to be examined, to a second position for examining the cavity (2),
**characterized in that**
- at least one movable video unit (21, 33, 40, 47) comprises an optical entrance window (23, 35, 41, 52), through which images of the cavity (2) can be taken and which faces the inside of the shaft (19), while the at least one movable video unit (21, 33, 40, 47) is in the first position and that,
- in the second position, the at least one movable video unit (21, 33, 40, 47) is splayed out from the shaft (19) by a pivoting motion (38, 45, 50),
- which transfers the at least one movable video unit from the first position to the second position,
- which is performed around a pivot axis (24, 44, 51) oriented in a transverse direction with respect to the longitudinal axis (20) of the shaft (19), and
- which increases the angle between the longitudinal axis (20) of the shaft (19) and a plane (A, B, D, H) that is perpendicular to the optical axis (Z) of the at least one movable video unit (21, 33, 40, 47).

2. The endoscope according to Claim 1,
wherein at least one stationary video unit (42, 49), whose position with respect to the shaft (19) is unchangeable, is disposed at the distal end (5) of the shaft (19).

3. The endoscope according to Claim 1 or 2,
wherein the video units (21, 33, 40, 42, 47, 49) are disposed at the same position along the longitudinal axis (20) of the shaft (19) in the first and/or second position of the at least one movable video unit (21, 33, 40, 47).

4. The endoscope according to any one of Claims 1 to 3,
wherein the optical entrance windows (23, 35, 41, 52) of the at least one movable video unit (21, 33, 40, 47) faces a lateral side of the shaft (19) in the first position of the at least one movable video unit (21, 33, 40, 47).

5. The endoscope according to any one of Claims 1 to 4,
- wherein at least two video units (21, 33, 40, 47) are movable and pivotably attached to the shaft (19) by links of the at least two movable video units (23, 35, 41, 52) and the shaft (19), and
- wherein optical entrance windows (23, 35, 41, 52) of the at least two movable video unit (21, 33, 40, 47) face the inside of the shaft (19) while the at least two video units (21, 33, 40, 47) are in the first position and
- wherein the at least two video units (21, 33, 40, 47) are splayed out from the shaft (19) by pivoting motions (38, 45, 50),
- which transfer the at least two video units (21, 33, 40, 47) from the first position to the second position,
- each of which is performed around a pivot axis (24, 44, 51) oriented in a transverse direction with respect to the longitudinal axis (20) of the shaft (19), and
- which increase the angles between the longitudinal axis (20) of the shaft (19) and planes (A, B, D, H), that are perpendicular to the optical axis (Z) of the at least two movable video units (21, 33, 40, 47).

6. The endoscope according to Claim 5,
wherein the optical entrance windows (23, 35, 41, 52) of the at least two movable video units (21, 33, 40, 47) are facing each other in the first position of the movable video units (21, 33, 40, 47).

7. The endoscope according to Claim 5 or 6,
wherein each pivoting motion of the movable video units (21, 33, 40, 47) is performed around an associated pivot axis (24, 44, 51) and that the pivot axes (24, 44, 51) are different.

8. The endoscope according to any one of Claims 1 to 7,
wherein the actuating elements (25-30) comprises a stop (29) against which a counterpart (27) abuts in the second position for adjusting the position of the at least one movable video unit (21, 33, 40, 42, 47, 49).

9. The endoscope according to any one Claims 1 to 8,
wherein the link between the at least one movable video unit (21, 33, 40, 47) extends between the proximal end of the at least one movable video unit (21, 33, 40, 47) and the shaft (19).

10. The endoscope according to any one of Claims 1 to 9,
wherein the the shaft (19) and/or any one of the video units (21, 33, 40, 42, 47, 49) is provided with a an illumination device (39) and/or a component (46) for retrieving spatial coordinates of the cavity (2) to be examined or an object (14) contained in the cavity (2).

11. The endoscope according to any one of Claims 1 to 10,
wherein the at least one movable video (21, 33, 40, 47) unit partially covers the distal end (5) of the endoscope in the first position.

12. An endoscope system comprising:
- a image processing unit (54) arranged to be connected to at least two video units (21, 33, 40, 42, 47, 49) of an endoscope (4);
- a display (11) connected with the image processing unit (54) and arranged for displaying a three-dimensional view (12) of the cavity(2) to be examined and of objects (14) contained in the cavity (2),
**characterized in that**
the image processing unit (54) is arranged to be connected to the video units (21, 33, 40, 42, 47, 49) of the endoscope (4) according to any one of claims 1 to 11.

13. The endoscope system according to Claim 12,
wherein the display (11) is an autostereoscopic display, a holographic display or a display system using a binocular filter device.

14. The endoscope system according to Claim 12 or 13,
wherein the endoscope system is further provided with a data reduction unit (55) for retrieving spatial coordinates of the cavity (2) to be examined and of objects (14) contained within the cavity (2), wherein the data reduction unit(55) is arranged to process images recorded by the video units (21, 33, 40, 42, 47, 49) and/or additional data provided by further components (46) located in the video units (21, 33, 40, 42, 47, 49) and/or the shaft (19) of the endoscope (4).

15. The endoscope system according to Claim 14,
wherein, in the view (12), a representation (13) of an object (14) within the cavity (2) or limiting the cavity (2) is merged with an three-dimensional model (18) of the object (14) obtained from another modality, wherein the present location, orientation, and/or dimensions of the object (14) as seen from the distal end (5) of the endoscope (4) are obtained from the data reduction unit (55) and wherein the location, orientation, and/or dimensions of the model (18) within the view (12) are adapted to the three-dimensional representation (13) of the object (14) depending on the present location, orientation, and/or dimensions of the object (14) obtained from the data reduction unit (55).
